(19)

**Europäisches Patentamt**
**European Patent Office**
**Office européen des brevets**

(11) **EP 4 272 762 A1**

(12) **EUROPEAN PATENT APPLICATION**
published in accordance with Art. 153(4) EPC

(43) Date of publication:
**08.11.2023 Bulletin 2023/45**

(21) Application number: **21915924.1**

(22) Date of filing: **09.08.2021**

(51) International Patent Classification (IPC):
**A61K 47/10** $^{(2017.01)}$     **A61P 31/14** $^{(2006.01)}$

(86) International application number:
**PCT/RU2021/000341**

(87) International publication number:
**WO 2022/146174 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.12.2020 RU 2020143732**

(71) Applicant: **Betuvaks Limited Liability Company Moscow, 121096 (RU)**

(72) Inventors:
• **ISAEV, Artur Alexandrovich**
  **Moscow, 127051 (RU)**

• **KRASILNIKOV, Igor Viktorovich**
  **Moscow, 119590 (RU)**
• **FROLOVA, Maria Evgenievna**
  **Moscow, 119602 (RU)**
• **KUDRYAVTSEV, Alexander Viktorovich**
  **Novgorod region, 607182 (RU)**
• **VAKHRUSHEVA, Anna Vladimirovna**
  **Vladivostok Primorsky Territory, 690005 (RU)**
• **IVANOV, Alexander Viktorovich**
  **Territory, g. Perm, 614007 (RU)**

(74) Representative: **Jeck, Anton**
  **Jeck, Fleck & Partner mbB**
  **Patentanwälte**
  **Klingengasse 2**
  **71665 Vaihingen/Enz (DE)**

(54) **METHOD OF OBTAINING BETULIN AS AN ADJUVANT IN A VACCINE AGAINST CORONAVIRUS SARS-COV-2**

(57) The invention relates to biotechnology, and specifically to a method for creating the adjuvant betulin, suitable for preparing a vaccine against coronavirus SARS-CoV-2. The method consists in sterilizing filtration of a solution of betulin in tetrahydrofuran through a nylon membrane with a pore diameter of 0.22 $\mu$m, decreasing the tetrahydrofuran content by adding a 25-fold volume of sterile 0.01 M tris-buffer (pH 9.0$\pm$0.1), and subsequently homogenizing by ultrasound until a homogeneous suspension results, forming spherical amorphous homogeneous particles suitable for binding proteins of the SARS-CoV-2 virus. The proposed technique makes it possible to produce betulin with high sterility and immunogenicity, which improves the quality of the vaccine against the coronavirus.

EP 4 272 762 A1

## Description

In December 2019, there was an outbreak of a severe acute respiratory infection called COVID-19 (CoronaVirus Disease-19) caused by the novel coronavirus SARS-CoV2 in China. Since its emergence, the virus has spread almost to all countries in the world. The World Health Organization announced that the outbreak had become a pandemic. The COVID-19 epidemic became the third epidemic caused by corona-viruses, after the epidemics of severe acute respiratory syndrome in 2002-2003 and the epidemic of the Middle East respiratory syndrome. According to the WHO, more than 65 million people had been infected with the novel coronavirus and 1.4 million people had died because of COVID-19 as of the beginning of December 2020. The ongoing COVID-19 epidemic poses a serious threat to humanity, including a direct impact on the daily lives of millions of people and a negative impact on the global economy.

No effective options of etiotropic or pathogenesis-based therapy for coronavirus infections have been developed so far. The main objectives of etiotropic and pathogenesis-based therapy is to reduce the viral load and/or decrease the severity of lung inflammation. Currently, there are no approved agents for etiotropic therapy for the SARS-CoV-2 infection, although there are quite a large number of candidate drugs tested in non-clinical studies. There are also pathogenesis-based and symptomatic treatments aimed at reducing the severity of systemic and lung inflammation, restoring blood oxygenation, fluid and electrolyte balance etc. Therefore, the main approach to combat COVID-19 should be timely vaccination.

There are no SARS-CoV-2 vaccines approved for clinical use, however, many candidate drugs have been developed. Candidate vaccines belong to the following types:

1) subunit vaccines (usually recombinant S-protein based vaccines or receptor-binding domain-based vaccines);

2) DNA vaccines based on genetic constructs encoding the full-length protein S or its receptor-binding domain;

3) vector-based vaccines that are based on non-pathogenic viruses (adenoviruses, Vaccinia viruses et al.), the genome of which includes one or several coronavirus genes;

4) inactivated whole-virion vaccines;

5) genetically engineered attenuated live vaccines based on wild-type viruses aimed at eliminating or inactivating virulence factors (inhibitors of the interferon response, mediators of cytopathic effects).

All listed vaccines can contribute to creating protective titers of neutralizing antibodies. In order to increase the efficacy of vaccination, it is advisable to introduce immunoadjuvants into the composition of vaccines.

The use of adjuvants may help increase the immunogenicity of vaccines in terms of the number of antigenically diverse strains, or during immunization of various populations including risk groups. Moreover, with a significant increase in immunogenicity due to the addition of an adjuvant to the vaccine, it becomes possible to switch to simple (single dose) immunization regimens, as well as to reduce the antigen dose. This is especially important for vaccines used during the pandemic, because with the same manufacturing capacity, more doses of the vaccine will be received, and, as a result, more people will be immunized.

Thus, the development of a safe and effective product manufactured in Russia (a vaccine against the coronavirus infection containing a corpuscular adjuvant) appears to be a strategically important aspect of protecting the population of the Russian Federation from the severe social and economic consequences of the pandemic, which can be controlled by prophylactic immunization.

### *List of figures*

Fig. 1 - Betulin structural formula

Fig. 2 - Chromatographic profile of THF in the CA sample after ultrafiltration

Fig. 3 - Size of spherical amorphous CA nanoparticles (SACANP)

Fig. 4 - Chromatographic profile of THF in the CA sample after ultrafiltration

Fig. 6. Chromatographic profile of a drug substance sample.

Fig. 7. Chromatographic profile of a vaccine test sample (supernatant fluid)

## Description of the invention.

We have developed a laboratory technique for producing a corpuscular adjuvant (CA) based on natural betulin for its subsequent use in the vaccine manufacturing. The object of development is a corpuscular adjuvant based on a natural pentacyclic triterpene - betulin (betulenol, betulinol, lupendiol). The drug is intended for use as an adjuvant of prophylactic vaccines.

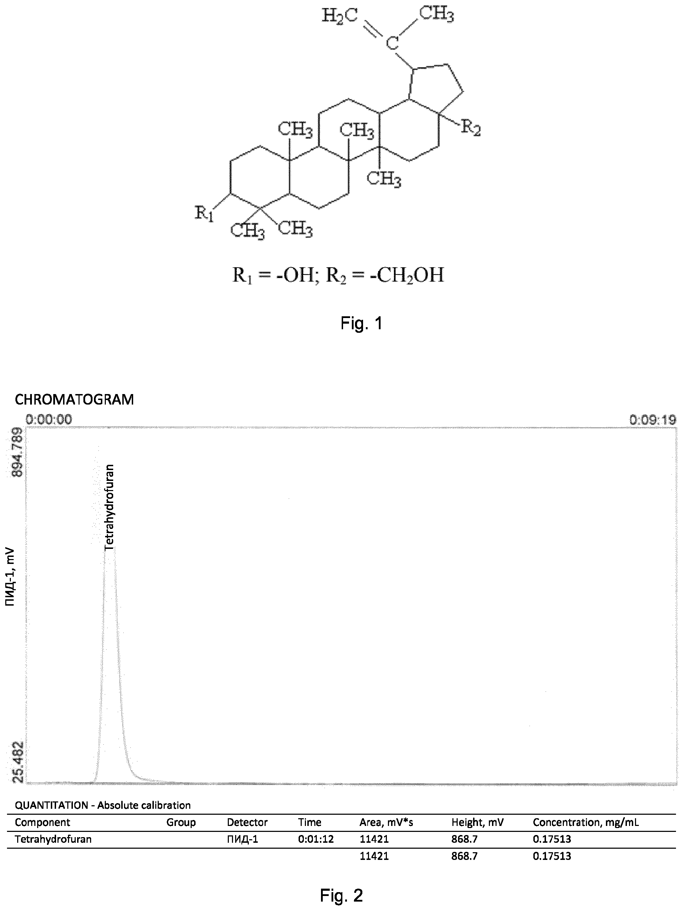

Betulin is a triterpene alcohol with two hydroxyl groups (Fig. 1). The solubility of betulin is less than 1 $\mu$g/mL, so the creation of a water-soluble form is very important. The search for such forms led to the creation of birch bark-based spherical amorphous nanoparticles.

Adjuvants used in the vaccine manufacturing, in accordance with the WHO guidelines and regulatory documents adopted in Russia, are subject to the following requirements: they must be sterile, pyrogen-free, and non-toxic. However, both the laboratory technique for the CA production developed at the Department of Biotechnology and Nanobiotechnology of Moscow State University of Fine Chemical Technologies named after M.V. Lomonosov and its scaled version do not include a sterilization stage [RU 2355423"Adjuvant"; A61K47/06; C08H5/04; published on May 20, 2009]. According to this method, the adjuvant is obtained by dissolving the commercially available dry birch bark extract in an organic solvent, tetrahydrofuran (THF), at a concentration of 2.5-5 g/L; a large amount of water is added to the specified solution (up to 25 volumes of water relative to the volume of the solution); after that, the solvent and most of the water are removed. A cryoprotector (e.g., sorbitol) is added to the resulting aqueous solution of nanoparticles and lyophilization is carried out. Before use, the adjuvants are dissolved in a phosphate buffer (pH 7.5), sonicated for 15 minutes, after which they are cooled.

The objective of the invention was to create a technique for the CA production from natural betulin that meets the requirements for vaccines for parenteral use in terms of sterility, pyrogenicity, immunogenicity and safety.

The technical result is the development of a method of production of corpuscular betulin to be used as an adjuvant in a SARS-COV2 vaccine using an optimal sterilization technique.

## Elaboration of the CA sterilization technique

At the first stages of our research, the search for the optimal CA sterilization technique that could be used at the final stages of the manufacturing process was carried out. Possible use of thermal and membrane sterilization techniques was evaluated for the purpose of producing a sterile adjuvant.

Steam sterilization was used as a thermal method under the following conditions: 120 °C - 0.5 atmosphere (0.5 kgf/cm2) - 10 minutes; 120 °C - 1.0 atmosphere (1.0 kgf/cm2) - 45 minutes. As a result of the conducted tests, the testing conditions of heat treatment were found to be unsuitable: after sterilization, large flake-like particles were formed that did not disintegrate with intensive stirring, which floated to the surface during prolonged storage. Heat treatment caused their aggregation by impairing the structure of amorphous nanoparticles.

In order to elaborate membrane sterilization techniques, tests were conducted to assess the possibility of using membranes made of nylon, polyethersulfone, and cellulose acetate. The experiments showed that particle absorption occurred on the surface of such filters: the filtered material above the membrane became transparent due to the formation of a particle deposit on it, while the filtration capacity of the membrane decreased. Therefore, these filter elements turned out to be unsuitable for sterilizing filtration of the CA.

Thus, CA membrane and heat sterilization techniques cannot be used at the final stages of the manufacturing process.

## Production of a sterile betulin solution in tetrahydrofurane as a basis for the CA manufacture.

To obtain an adjuvant that meets the requirements for products for parenteral use, we have proposed a technique where all stock solutions are to be sterilized at the initial stages of the CA production, and all further manufacturing process should take place in sterile conditions.

During the elaboration of the conditions of sterilizing filtration of the betulin solution in tetrahydrofuran (10 mg/mL), a NRG Pall N66+ nylon membrane with a pore size of 0.22 $\mu$m was used. This filter element is hydrophilic with a modified charge, so initially it seemed appropriate to evaluate its absorption capacity, which could negatively affect the finished product yield. For this purpose, the Contractor conducted experiments determining the content of betulin by HPLC before and after filtration, and the betulin absorption percentage was calculated thereafter (see Table 1).

Table 1. Membrane absorption capacity determination

| No. | Content of betulin prior to filtration, mg/mL | Content of betulin after filtration, mg/mL | Betulin adsorption, % |
|---|---|---|---|
| 1 | 10.021 | 9.810 | 2.11 |
| 2 | 10.518 | 10.336 | 1.73 |
| 3 | 10.250 | 10.009 | 2.35 |
| M±m | | | 2.06±0.31 |

As shown in Table 1, the membrane used has a low absorption capacity: the content of betulin in the solution before and after the filtration differs very slightly, only about 2% of the active substance was absorbed by the membrane. Therefore, the absorption properties of the NRG Pal N66+ filter element tested in experiments allow using it for the sterilizing filtration of the CA.

Next, it was necessary to demonstrate the chemical stability of the membrane potential relative to the organic solvent tetrahydrofuran (THF). For this purpose, the bubble point technique recommended by the methodical guidelines MY 42-51-19-93 of the Ministry of Health of the RF was applied. In accordance with the GMP requirements, when filtration systems are used, it is necessary to check the integrity of the membrane filter elements. According to MY 42-51-19-93, testing for the integrity should be conducted before and after each filtration procedure during the sterilization filtration. It should be noted that the impaired integrity of the filter element (membrane) can also occur during its sterilization, when the filter is exposed to high temperatures and high pressure. In experiments to determine the integrity of membranes before and after filtration of a THF-containing betulin solution using the "bubble point" method, a visual inspection of the membranes after the sterilizing filtration process was also carried out.

The bubble point test is carried out automatically after the end of filtration. When the entire volume of liquid has been filtered out, the gas flow used for the transfer should automatically stop as soon as the gas reaches the surface of the liquid-saturated membrane. There should be no gas bubbles in the glass receiver.

The experiments showed that the filter membrane maintained the excess pressure created in the filter system for 20 minutes, and no gas bubbles passed through the liquid layer. Visual inspection of the membranes after the filtration also confirmed the integrity of the filter element (membrane). The results demonstrated high chemical stability of the sterilizing membrane.

Therefore, the experiments that showed a low absorption capability of the NRG Pall N66+ sterilizing membrane relative to the betulin solution in THF and its stability to the effects of THF, have proven the possibility of using the membrane sterilization of the stock solution as part of the CA production technique.

*Producing a homogeneous CA dispersion*

According to the method, in order to obtain CA homogeneous dispersions to the sterile mixture of 1% betulin solution in THF, the volume of the sterile 0.01 M Tris buffer (pH-9.0±0.1) that was 25 times the volume of the mixture was added using a peristaltic pump while constantly stirring for 15 min using a propeller-style magnetic stirrer. After adding the buffer, the content of THF in the solution was 3.85%. Then the resulting suspension was sonicated at 35 kHz three times, each time for 30 seconds. For this purpose, the container with the resulting suspension was sonicated in an ultrasonic bath. As a result, a white homogeneous suspension without any conglomerates or inclusions was obtained. The homogeneity of the suspension was monitored based on light scattering by sequentially treating it for 30 seconds to obtain two identical particle distribution spectra in the range of 100-150 nanometers.

*Ultrafiltration. Elaboration of the purification technique*

At the next step, the CA dispersion purification technique was elaborated. In order to remove toxic THF, hollow-fiber ultrafiltration was applied with a nominal molecular weight cut-off of 100 or 300 KDa in the filtration mode. At the first stages of ultrafiltration, with a rapid decrease in the THF concentration, partial adsorption of the CA occurred on the membranes of the equipment, and the filtration rate decreased slightly, while the finished product yield became lower. Therefore, it was necessary to elaborate the ultrafiltration parameters that prevent the absorption on the CA membranes and ensure effective removal of THF. The experiments conducted to elaborate the conditions of the purification procedure showed that a two-fold or a three-fold dilution of the stock CA dispersion had to be used to prevent absorption. In this case, the ultrafiltration rate was 1.0-1.2 L/min, and the pressure value had to be within the range of 0.6-0.8 atm. For diafiltration, the volume of 0.01M Tris-buffer (pH = 9.00) that was 10 times higher was used to increase the adjuvant output The method according to claim 1 characterized in that after each volume of the diafiltration buffer solution, the reverse flow is turned on, followed by sonication of the suspension for 3 minutes at 35 kHz in order to increase the adjuvant yield.

Then the dispersions were concentrated until the concentration of betulin reached 1.0-2.0 mg/mL. Betulin concentration was determined by HPLC. Following the ultrafiltration, the residual amount of THF was monitored in the CA using gas-liquid chromatography (see Fig. 2).

The content of THF after ultrafiltration was shown to comprise 0.176±0.009 mg/mL.

Therefore, the elaborated conditions of the ultrafiltration process ensure effective removal of toxic THF.

### *Determination of the resulting CA particle characteristics*

The resulting CA was a white to yellowish-white dispersion separating during storage into a colorless clear supernatant fluid and a white to yellowish-white loose precipitate (which completely breaks down as a result of shaking).

First, we evaluated the finished product in terms of sterility by direct inoculation to a culture medium. 1 mL of the adjuvant with the CA concentration of 1 mg/L (after its reconstitution from a freeze-dried powder) was added to test tubes with a thioglycolate medium. The test tubes were incubated at 32.5±2.5°C and 22.5±2.5°C. After 14-day incubation, the culture medium in the test tubes remained transparent, i.e. there was no microbial growth. Therefore, the resulting freeze-dried CA samples were proven to be sterile.

For pyrogen testing, CA samples were administered intravenously to rabbits at a dose of 200 $\mu$g/kg body weight. The maximum sum of temperature changes in three rabbits was 0.9°C (see Table 2) indicating that the CA was pyrogen-free.

Table 2. CA pyrogen testing

| Rabbit No. | Rabbit weight, kg | Temperature prior to administration, °C | Temperature in an hour, °C | Temperature in two hours, °C | Temperature in three hours, °C | Sum of maximum temperature changes, °C |
|---|---|---|---|---|---|---|
| 1 | 2.75 | 39.4 | 39.3 | 39.5 | 39.5 | 0.9 |
| 2 | 2.65 | 39.3 | 38.9 | 39.0 | 39.2 | |
| 3 | 2.50 | 39.5 | 39.1 | 39.2 | 39.3 | |

Therefore, the developed technique allows obtaining sterile and pyrogen-free CA, which meets the requirements for the products for parenteral use and allows using them as vaccine adjuvants.

The main characteristic of nanoparticle-based adjuvants is the size and zeta potential, since their adjuvant capability depends on the size and charge of the particles.

As was demonstrated by many authors, it is the particle size that plays a crucial role in the adjuvant efficacy. The submicron-size particles have been shown to be the most effective immunological adjuvants. According to P.L. Mottram, Type 1 and 2 immunity following vaccination is influenced by nanoparticle size: formulation of a model vaccine for respiratory syncytial virus / P. Mottram [et al.] // Mol. Pharm. - 2006. - Vol. 4, No. 1 - P. 73-84, the particle size <500 nm was the optimal one to ensure the particle absorption by macrophages.

During the stage of elaboration of the CA production technique, the zeta potential and the particle size were determined. The zeta potential was determined by electrophoretic light scattering based on changes in the particle distribution in the electrical field using a Zetasizer Nano apparatus (Malvern). The zeta potential is an electrical potential that occurs when particles move between the adsorption layer of ions located on the surface of the particles and the diffusion layer of ions of the medium surrounding the particle. CA particle size was determined by laser light scattering at 170° and $\lambda$=633 nm using a Zetasizer Nano ZS apparatus (Malvern). The method of particle size determination is based on light-scattering spectroscopy. The method is absolute and does not require pre-calibration using reference standards. Laser light scattering is a method for measuring the average velocity of dispersed particles by analyzing dynamic fluctuations in the light scattering intensity, i.e. determining the spectral density or time correlation function of scattered light intensity. Chaotic Brownian motion of dispersed particles leads to macroscopic fluctuations of their local concentration, which, in turn, causes local fluctuations of the refractive index of the medium. When a laser beam, which is a flat, monochromatic, linearly polarized wave, passes through such a medium, part of the light will be scattered on these local inhomogeneities. Fluctuations in the scattered light intensity will correspond to fluctuations in the local concentration of dispersed particles, which will allow measuring the diffusion coefficient of dispersed particles in a fluid. Since the diffusion coefficient of dispersed particles is uniquely related to the particle size, laser light scattering is essentially a method for measuring the size of such submicron particles. The particle size is calculated by the Stokes-Einstein formula, which relates the particle size to their diffusion coefficient and the viscosity of the fluid.

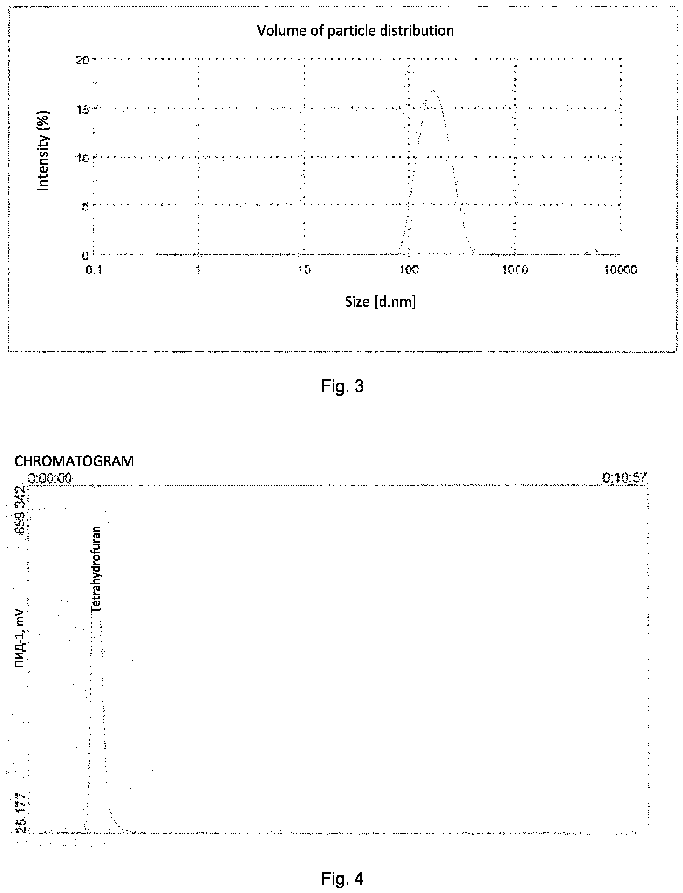

A can be seen in Fig. 3, the average size of particles in the resulting CA samples is 160-180 nm. The zeta-potential value was equal to minus 44.3 mV. The negative charge may contribute to better binding to the antigen.

Therefore, the proposed technique can be used to obtain particles that effectively bind the antigen and have an adjuvant effect.

***Methods of quality control for CA samples***

***Determination of the betulin concentration in CA dispersions***

During the process of elaboration of the CA production technique and assessment of the finished product, one of the important control parameters is the concentration of betulin. The simplest way to determine the concentration of dispersion by estimating light scattering cannot provide accurate information, since the parameter depends not only on the number of particles, but also on their size and shape. Minor changes in the size and shape of nanoparticles can cause significant light scattering differences.

These experiments helped elaborate an original technique of determining the betulin concentration using high-performance liquid chromatography (HPLC). HPLC is a method that has been actively developing over the past decades, has been shown to be one of the most universal techniques for separation and pharmacopoieal analysis of drug substances and drug products.

HPLC can be used for separation of a wider variety of substances compared to gas chromatography since most substances are not volatile, and many biologically active substances are unstable at high temperatures.

Of all the available HPLC variants, reversed-phase HPLC is the most commonly used methods. The method is characterized by simplicity and versatility, and in many cases by the simplicity of the absorption mechanism and the predictability of the substance behavior based on their structure. Currently, most tests are carried out on non-polar mobile phases using ultraviolet, fluorometric, electrochemical, and mass spectrometry detectors.

HPLC is characterized by its high selectivity and sensitivity, along with versality, which allows conducting qualitative and quantitative testing of substances and finished dosage forms under the same conditions.

It has been shown that the betlin identification should be carried out using its solution in ethanol. The absorption spectrum of the resulting solution shows a maximum in the ultraviolet region of the spectrum at 210 nm, which was chosen as the working wavelength for the assay of betulin by HPLC. The use of THF as a solvent is unacceptable as it is not transparent at this wavelength.

Chromatography was carried out using a LC-20 Prominence chromatographic system (Shimadzu, Japan) equipped with a diode array spectrophotometric detector, a mobile phase supply pump, a degasser, an automatic injector with the ability to maintain the temperature of samples at +4°C, a thermostatically controlled column compartment and a computer with software for collecting and processing chromatographic data.

The parameters of separation of the test betulin solution were evaluated using two analytical columns: Kromasil 100-C18 (5 $\mu$m, 150 x 4.8 mm) and Kromasil 100-C8 (5 $\mu$m, 150 x 4.8 mm) manufactured by AkzoNobel (Netherlands). The experiment showed that selective separation of the main substance and the most closely eluting peaks was observed using column C18, which was applied in further tests.

When choosing the composition of the mobile phase, the following components were considered: methanol, acetonitrile, and water.

The experiments showed that the use of mobile phases with the aqueous content of 30-40% (by volume) in the isocratic elution mode resulted in a significant peak widening and insufficient resolution of the test substance peak as well as the closest peak (Rs did not exceed 1.3).

Furthermore, the betulin concentration of 1.0 mg/mL was associated with a widening of the frontal edge of the peak resulting in a loss of the betulin separation effectiveness. Thus, the optimal working concentration of betulin was determined as 0.2 mg/mL.

During further tests, satisfactory betulin separation parameters in the isocratic elution mode were achieved. The potent component of the eluent is acetonitrile.

To evaluate the dependence of the betulin peak area from its concentration in the test solution, an experiment was conducted using model mixtures of the betulin drug substance in the ratio equivalent to that in the CA freeze-dried powder. The range of betulin concentrations was 0.16 to 0.24 mg/mL.

To plot the calibration curve, a reference standard (RS) solution was prepared. About 10 mg (accurately weighed quantity) of the betulin RS was placed in a 50-mL volumetric flask, and 10 mL of the ethanol solvent was added; after that, the volume of the solution was brought to the mark with the same solvent, and mixed. The resulting solution was filtered through a Millipore filter with a pore size of 0.45 $\mu$M (or equivalent) discarding first 2 mL of the filtrate (0.2 mg/mL).

Preparation of standard solutions for plotting the calibration curve.

Immediately before the test, five standard solutions of betulin were prepared as shown in Table 3.

Table 3. Concentrations of betulin solutions

| Betulin concentration, mg/mL | Betulin RS solution volume (0.4 mg/mL), μL | Alcohol volume, μL |
|---|---|---|
| 0.16 | 400 | 600 |
| 0.18 | 450 | 550 |
| 0.20 | 500 | 500 |
| 0.22 | 550 | 450 |
| 0.24 | 600 | 400 |

To prepare the test solution, one vial with an adjuvant was taken; the contents of the vial were diluted in 10.0 mL of ethanol (about 10 mg/mL of betulin). The resulting solution was placed in a dark place for two hours, sonicated for 10 min, cooled down and mixed. A portion of the resulting solution was filtered through a Millipore filter with a pore size of 0.45 μM (or equivalent) discarding the first 2 mL of the filtrate. 5.0 mL of the filtrate was placed in a 25.0 mL volumetric flask, the volume of the solution was brought to the mark with ethanol, and mixed (about 0.2 mg/mL).

The chromatographic system was conditioned prior to the assay by passing the mobile phase for at least 30 min until a stable baseline was obtained.

Chromatography of 20 μL of the solvent and the RS sample was sequentially performed, recording at least three chromatograms for each dilution. A calibration curve was plotted. Then chromatography of 20 μL of the test solution was performed and at least three chromatograms were recorded. The run time for the test solution was twice the principal peak retention time.

The content of betulin in the test sample in milligrams (X) was calculated by the following formula:

$$X = \frac{(S-b)*10*25}{a*5}$$

where: a is the tangent of the angle inclination of the linear regression to the X axis;

b is the coordinate of the intersection point of the linear regression with the Y axis;

S = test sample peak area.

### THF concentration determination

The technique of the CA production proposed by the Contractor comprises the ultrafiltration stage to remove THF, which is a weakly toxic substance (LD50 = 2.3 g/kg; MPC = 100 mg/m$^3$). The quality of ultrafiltration purification was controlled based on the content of THF in the dispersion and the permeate. The residual concentration of the solvent should also be determined in the finished product. For this purpose, we used gas fluid chromatography.

The data obtained when testing the samples at the stage of ultrafiltration in the permeate (1) and concentrate (2) are shown in Table 4. The amount of THF in the stock sample prior to ultrafiltration was 3.85%.

Table 4. THF concentration in samples

| Sample | THF concentration, mg/mL n=3 |
|---|---|
| 1 | 0.176±0.009 |
| 2 | 0.122±0.004 |

The analysis of the results showed that the ultrafiltration stage helps reduce the residual concentration of THF to the residual concentration of THF to as low as 0.122 mg/mL (see Fig. 4). Therefore, the administration of a vaccine dose (200 μg in 0.5 mL) delivers the dose of THF that is significantly less than the LD50 into the body. The elaborated ultrafiltration technique ensures a significant decrease in the THF concentration.

*Photometric dispersion parameter determination*

In order to standardize the CA manufacturing process, it is reasonable to use the photometric dispersion (PHD) parameter. The method for determining the PHD is based on the fact that, within a fairly wide range, the light scattering by dispersions directly depends on the concentration, particle size and their shape and consists in determining the absorbance at two wavelengths, followed by calculating the index of dispersion; small changes in these parameters lead to a significant change in light scattering. By determining the PHD, it is possible to effectively monitor the manufacturing process and control the particle production.

The analysis of 6 CA series showed that standard dispersions are obtained if all parameters of the manufacturing process are met (see Table 5).

Table 5. Photometric dispersion (PHD) parameter,
SACANP

| Measurement No. | PHD |
|---|---|
| 1 | 0.980 |
| 2 | 0.995 |
| 3 | 0.982 |
| 4 | 1.031 |
| 5 | 0.996 |
| 6 | 1.007 |
| M±m | 0.577 ± 0.05 |

The conducted experiments showed that the PHD for the CA at a concentration of 0.5 mg/mL was 0.577 ±0.05.

Thus, the PHD can be used to standardize the process of producing and characterizing a finished natural betulin-based CA adjuvant.

Then, the laboratory technique of obtaining the recombinant fluid SARS-CoV-2 vaccine, 10, 40 $\mu$g/mL using the CA was developed.

The first stage consisted in the development of a technique of recombinant RBD-FC protein created based on the SARS-CoV-2 virus genome.

The second stage included the selection of optimal concentrations of the antigen component and the content of the adjuvant in the vaccine in order to ensure the vaccine dose optimization.

The third stage included optimization of the process of combining the antigen and the adjuvant to formulate a ready-to-use vaccine.

The drug substance for a SARS-CoV-2 vaccine was produced by recombinant technologies. Drug substance is a highly purified recombinant receptor-binding domain (RBD) of the SARS-CoV-2 spike protein (S protein).

The recombinant RBD protein technology includes the following stages: cloning of the RBD gene sequence into an expression vector, induction of cells of the producer strain, culture of producer cells, precipitation of cells from the culture medium by centrifugation, lysis and destruction of cells, separation by differential centrifugation of proteins of an easily soluble fraction and insoluble proteins, purification of recombinant proteins using affinity chromatography on an agarose carrier, containing the protein A attached by a covalent bond, and sterilizing filtration. The developed technique allows purifying the recombinant protein from the biomass of the producing strain and further scale-up of the process to industrial scale.

*Vaccine technology development*

As a result of conducted experiments, the following composition of one dose (0.5 mL) was proposed:

| Component | Amount per dose (0.5 mL) | Intended use |
|---|---|---|
| *Active substances:* | | |
| SARS-CoV-2 receptor-binding domain (RBD) of the spike protein | 5 $\mu$g or 20 $\mu$g | Antigen |

(continued)

| Excipients: | | |
|---|---|---|
| Natural betulin-based corpuscular adjuvant | 200 μg | Adjuvant |
| Tris-HCl 0.05 M containing sodium chloride 0.15 M | q.s. to 0.5 mL | |

**Examples of vaccine production.**

Studies were conducted to elaborate and upgrade the laboratory technique of the vaccine manufacturing. Therefore, the laboratory guideline was developed for the product "SARS-CoV-2 vaccine, recombinant fluid vaccine, 10 μg/mL".

**Example 1.** Sterile recombinant receptor-binding domain (RBD) of the SARS-CoV-2 spike protein (RBD antigen) was added to beforehand prepared sterile adjuvant in Tris-HCl buffer. The adjuvant was prepared in the same container placed in an ultrasonic bath. Sterile betulin solution in THF was fed to the container with sterile buffer solution while sonicating at 35 KHz for 10 min. After the betulin solution got into the container, it was stirred, and the resulting suspension underwent ultrafiltration to remove THF; after the end of ultrafiltration, the suspension was sonicated at KHz for 3 min. After that, a sterile recombinant antigen dissolved in Tris-HCl buffer was supplied into the container so that the final concentration of the RBD antigen was either 40 μg/mL or 10 μg/mL, and the concentration of the adjuvant was 400 μg/mL. The solution was thoroughly stirred, and the container was placed in a shaker into the refrigerator for 3 hours. Later, the bottles with the RBD antigen were stored in a refrigerator at 2 to 8 °C. The resulting vaccine bulk was poured into 0.5-mL vials. Series of vaccines P1 40 μg/mL (n=905) and 10 μg/mL (n=840) were obtained for nonclinical studies.

**Example 2.** 200 mL of the solution was supplied into a 5.5-L container with a sterile buffer solution 0.01 M (pH=8.8). 2% betulin solution in THF was sonicated for 10 min at 40 KHz. Then ultrafiltration of the obtained suspension was carried out using a buffer solution and membranes with a cut-off value of 100 KDa. After each volume of the diafiltration buffer solution, the reverse flow is turned on, followed by sonication of the suspension for 3 minutes at 35 kHz in order to increase the adjuvant yield. The volume of suspension was brought to 5 L. 5 L of the antigen solution consisting of a protein (SARS-CoV-2 nucleoprotein (N)) and RBD of the same virus in 0.05 M Tris-HCl buffer (pH = 7.6) containing 0.15 M NaCl were added while stirring vigorously. The final antigen concentration in the suspension was 10 μg/mL, and the adjuvant concentration was 400 μg/mL.

Then the container with the suspension was placed into a refrigerator (+4 C), and poured in the amount of 0.5 mL into vials one day later.

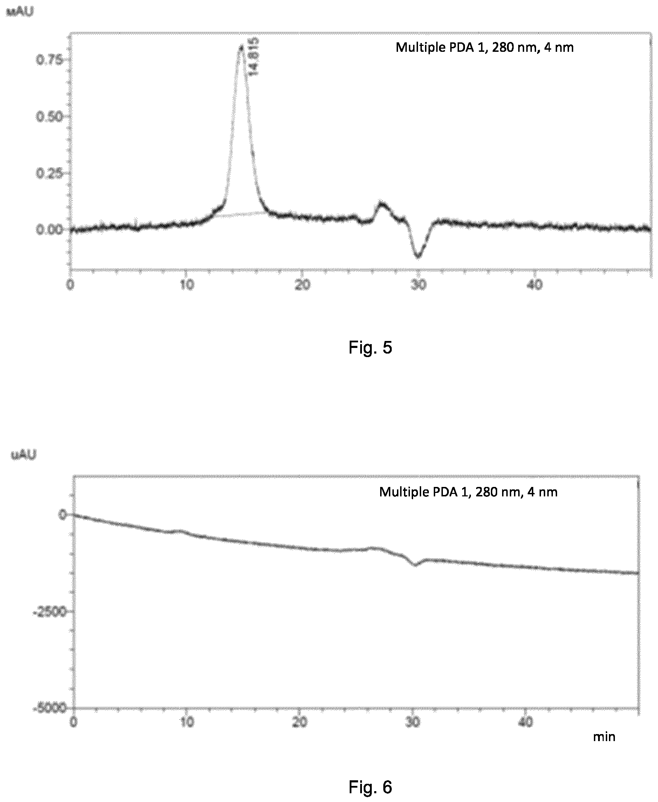

The assessment of the completeness of vaccine adsorption was carried out by HPLC using a liquid chromatography apparatus Shimadzu LC-2010 (Japan) and an analytical column Superdex 200 Increase 10/300GL. The test sample of the vaccine (0.5 mL) was centrifuged at 4,000 rpm for 30 min. The presence of the RBD in the supernatant fluid was determined based on the presence of a peak with the retention time of the main component that was close to the retention time of the peak of the reference product (RBD-Fc substance). Fig. 5 and 6 show chromatographic profiles of the drug substance and the test sample of the vaccine.

The absence of a peak with the retention time of 14.8 min in the vaccine sample indicate the completeness of the antigen absorption on the betulin adjuvant.

*Vaccine potency evaluation*

The goal of the study was to conduct a comparative assessment of the vaccine immunogenicity in animal tests. During the study, the production of antibodies in Balb/c mice weighing 12-14 g of both sexes immunized with experimental vaccines was tested. The test products in the amount of 0.5 mL were administered twice (according to the 0-14 regimen) in the peritoneal cavity using 1-mL syringes. Similarly, the control animal groups were injected with 0.9% sodium chloride. Blood sampling was carried out on Day 14 after a single-dose immunization and on Day 14 after a double-dose immunization. The antibodies were determined in sera using ELISA.

The potencies of a vaccine containing only RBD (in two dosages: 5 and 20 μg of the recombinant protein per dose (0.5 mL), N+RBD vaccine in a dosage of 5 μg antigen per dose (0.5 mL) and a vaccine without an adjuvant were studied.

Data on the number of animals, vaccines and their immunization are presented in Table 6.

Table 6. Number of animals required for the determination of the level of specific antibodies

| No. | Product name | Number of animals |
|---|---|---|
| 1 | RBD vaccine with an adjuvant, 10 μg/mL | 16 |
| 2 | RBD vaccine with an adjuvant, 40 μg/mL | 16 |
| 3 | RBD vaccine without an adjuvant, 10 μg/mL | 10 |
| 4 | RBD vaccine without an adjuvant, 40 μg/mL | 10 |
| 5 | N+RBD vaccine with an adjuvant, 10 μg/mL | 10 |
| 6 | RBD vaccine without an adjuvant, 10 μg/mL | |
| 7 | Non-vaccinated mice | 8 |
| Total number of mice | | 70 |

Sera obtained from the blood of mice (individually from each animal) were tested for the presence of antibodies to the SARS-CoV-2 antigen using an experimental test system-based immunoassay (ETIA). Commercially available reagents were used to construct the experimental test system: anti-mouse IgG (H+L) Strong Zyme HRP Conjugate (SDT, Germany); 3,3',5,5'-tetramethylbenzidine (Chema, Russia) as a chromogen; protein-salt solution as a blocker; 5% sulfuric acid solution as a stop reagent. Immunoenzymatic assay is carried out on polystyrene plates (Greiner bio-one, Germany). 1 μg of the SARS-CoV-2 antigen was applied to the substrate and adsorbed during a day. The results of ELISA were recorded on a PR 2100 spectrophotometer (Sanofi Diagnostics Pasteur, France) at two wavelengths (450/620 nm).

Table 7. Immunogenicity of the vaccine with an adjuvant in experiments on mice (geometric mean titer)

| Sample name (drug form) | Antibody level (geometric mean titer, confidence interval) x $10^{3*}$ | |
|---|---|---|
| | Immunization 1 | Immunization 2 |
| Vaccine with an adjuvant, 10 μg/mL | 1.7 [0,6-5.0] | 18.0 [10.4-31.0] |
| Vaccine with an adjuvant, 40 μg/mL | 3.2 [1.7-5.9] | 28.7 [12.2-35.2] |
| Vaccine without an adjuvant, 10 μg/mL | 0.9 [0.4-2.4] | 4.3 [2.7-16.9] |
| Vaccine without an adjuvant, 40 μg/mL | 0.8 [0.3-2.0] | 3.5 [1.0-12.5] |
| Vaccine with an adjuvant, 10 μg/mL | 1.4 (0.7-4.8) | 23.6 (15.3-30.7) |
| Vaccine without an adjuvant, 10 μg/mL | 0.8 (0.4-2.7) | 5.1 (2.3-11.8) |
| * - value that is inverse to the dilution | | |

Thus, immunization of animals with a vaccine with an adjuvant contributes to an increase in the antibody titer, while it should be noted that the product, which includes an adjuvant, has a more pronounced effect.

**Determination of the adjuvant (CA) content.**

The test was carried out using reversed-phase HPLC in accordance with the Russ. Pharm., general monograph ОФС.1.2.1.2.0005.15. The test was carried out using a Shimadzu LC-2010 liquid chromatography apparatus (Japan) and a Kromasil 300-5C8 250 x 4.6 mm analytical column with a particle size of 5 μm, AkzoNobel (Netherlands) in the isocratic mode of elution and detection at 210 nm.

Table 8. The results of determining of the betulin content in a vaccine with an adjuvant

| Product | Betulin content, μg per dose (0.5 mL) |
|---|---|
| Vaccine with an adjuvant, 10 μg/mL | 220 |
| Vaccine with an adjuvant, 40 μg/mL | 205 |

Thus, the conducted tests allowed developing the draft specifications for the product "SARS-CoV-2 vaccine, recombinant fluid, 10 and 40 μg/mL, which met all the requirements stated in the Russ. Pharm. and related to this product class. Draft specifications are shown in Table 9.

Table 9. CoviVax vaccine specifications

| No. | Parameters | Methods (control tests) | Requirements according to the ND |
|---|---|---|---|
| 1. | Appearance | Visual inspection | White to yellowish-white homogeneous dispersion dividing into a colorless transparent solution and a white to yellowish-white loose precipitate during storage. |
| 2. | Identification | Enzyme-linked immunosorbent assay (ELISA) | The result of the reaction for the detection of recombinant SARS-CoV-2 receptor-binding domain (RBD) of the spike protein must be positive |
| 3. | Particulate matter | Visual inspection, Russ. Pharm., general monograph ОФС. 1.4.2.0005.18 | Should meet the requirements |
| 4. | Sedimentation stability | Visual inspection, Russ. Pharm., general monograph ОФС. 1.4.1.0014.15 | Dispersion of the product that is formed during shaking should not layer within 2.5 min |
| 5. | Syringeability | Visual inspection, Russ. Pharm., general monograph ОФС. 1.4.1.0014.15 | Dispersion of the product formed during shaking should freely pass into the needle No.0840 |
| 6. | pH | Potentiometry, Russ. Pharm., general monograph ОФС. 1.2.1.0004.15 | From 7.0 to 7.6 |
| 7. | Extractable volume | Physical method, Russ. Pharm., general monograph ОФС. 1.2.1.0004.15 | Should be not less than the nominal one |
| 8. | Total protein | Spectrophotometry, Russ. Pharm., general monograph ОФС.1.8.2.0010.18 | From 4.0 to 5.0 μg per dose (0.5 mL) |
| 9. | Sterility | Direct inoculation or membrane filtration technique, Russ. Pharm., general monograph ОФС.1.2.4.0003.15 | The dispersion must be sterile |
| 10. | Pyrogenicity | Biological method, Russ. Pharm., general monograph ОФС. 1.2.4.0005.15 | The dispersion must be pyrogen-free |
| 11. | Abnormal toxicity | Biological method, Russ. Pharm., general mono-graph ОФС. 1.2.4.0004.15 | The dispersion must be non-toxic |
| 12. | Potency | Enzyme-linked immunosorbent assay (ELISA) | Should cause the production of antibodies in at least 80% of animals (mice). |
| 13. | Betulin adjuvant | HPLC, Russ. Pharm., general monograph ОФС. 1.2.1.2.0005.15 | from 160 to 240 μg per dose (0.5 mL) |
| 14. | Packaging | | 0.5 mL (1 dose) or 2.5 mL (5 doses) in vials according to the ОСТ 64-2-485-85 or ТУ 9462-002-11068395-2005, or imported ones that meet the ISO 9187-1 requirements. |
| 15. | Labeling | | In accordance with ND |

(continued)

| No. | Parameters | Methods (control tests) | Requirements according to the ND |
|---|---|---|---|
| 16. | Storage | | According to CΠ 3.3.2.3332-16 at 2 to 8 °C in a place protected from light. Do not freeze. |
| 17. | Shipping | | According to CΠ 3.3.2.3332-16 at 2 to 8 °C. Do not freeze. |
| 17. | Shelf life | | Being determined |

Therefore, it has been demonstrated that the vaccine comprising a betulin-based corpuscular adjuvant has a high antigenic activity.

**Claims**

1. A method of production of betulin as an adjuvant in a SARS-COV2 vaccine envisaging the use of sterilizing filtration of the betulin solution in tetrahydrofuran through a nylon membrane with a pore size of 0.22 μm, a decrease in THF concentration by adding the volume of sterile 0.01 M Tris buffer (pH-9.0±0.1) that is 25 times the volume of the mixture using a peristaltic pump while constantly stirring for 15 min using a propeller-style magnetic stirrer, followed by ultrasonic homogenization at 35 kHz for 10 min until a homogenous suspension is obtained and spherical amorphous homogeneous particles suitable for the SARS-CoV-2 proteins are produced.

2. The method according to claim 1 is **characterized in that** ultrafiltration is carried out using tangential ultrafiltration with hollow fibers or filters with a molecular weight cut-off of 100-300 kDa.

3. The method according to claim 1 **characterized in that** the suspension of the corpuscular adjuvant is sonicated at 35-40 kHz after the ultrafiltration to obtain a homogeneous suspension.

4. The method according to claim 1 **characterized in that** after each volume of the diafiltration buffer solution, the reverse flow is turned on, followed by sonication of the suspension for 3 minutes at 35 kHz in order to increase the adjuvant yield.

R₁ = -OH; R₂ = -CH₂OH

Fig. 1

CHROMATOGRAM

QUANTITATION - Absolute calibration

| Component | Group | Detector | Time | Area, mV*s | Height, mV | Concentration, mg/mL |
|-----------|-------|----------|------|-----------|-----------|----------------------|
| Tetrahydrofuran | | ПИД-1 | 0:01:12 | 11421 | 868.7 | 0.17513 |
| | | | | 11421 | 868.7 | 0.17513 |

Fig. 2

## Volume of particle distribution

Fig. 3

CHROMATOGRAM

Fig. 4

Fig. 5

Fig. 6

## INTERNATIONAL SEARCH REPORT

| | International application No. |
|---|---|
| | PCT/RU 2021/000341 |

**A.   CLASSIFICATION OF SUBJECT MATTER**
A61K 47/10 (2017.01); A61P 31/14 (2006.01)

According to International Patent Classification (IPC) or to both national classification and IPC

**B.   FIELDS SEARCHED**

Minimum documentation searched (classification system followed by classification symbols)
A61K, A61P, B82B, B82Y

Documentation searched other than minimum documentation to the extent that such documents are included in the fields searched

Electronic data base consulted during the international search (name of data base and, where practicable, search terms used)
EAPATIS, Espacenet, PatSearch (RUPTO internal), USPTO, PATENTSCOPE, eLIBRARY, ScienceDirect, Google Patents, Google Scholar

**C.   DOCUMENTS CONSIDERED TO BE RELEVANT**

| Category* | Citation of document, with indication, where appropriate, of the relevant passages | Relevant to claim No. |
|---|---|---|
| A | RU 2545717 C1 (OBSCHESTVO S OGRANICHENNOI OTVETSTVENNOSTJU OOO  "NANOLEK") 10.04.2015, p. 5, lines 25-28, p. 7, lines 11-15, examples, the claims | 1-4 |
| A | RU 2545714 C1 (OBSCHESTVO S OGRANICHENNOI OTVETSTVENNOSTJU "RAZVITIE BIOTEKHNOLOGY") 10.04.2015, examples, the claims | 1-4 |
| D, A | RU 2355423 C1 (OBSCHESTVO S OGRANICHENNOI OTVETSTVENNOSTJU "BEREZOVYI MIR") 20.05.2009, examples, the claims | 1-4 |

| ☐ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 October 2021 (13.10.2021) | 02 December 2021 (02.12.2021) |

| Name and mailing address of the ISA/ RU | Authorized officer |
|---|---|
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2355423

**Non-patent literature cited in the description**

- **P.L. MOTTRAM ; P. MOTTRAM.** Type 1 and 2 immunity following vaccination is influenced by nanoparticle size: formulation of a model vaccine for respiratory syncytial virus. *Mol. Pharm.*, 2006, vol. 4 (1), 73-84